(19) Europäisches Patentamt
European Patent Office
Office européen des brevets

(11) Veröffentlichungsnummer: **0 221 854 B1**

(12) **EUROPÄISCHE PATENTSCHRIFT**

(45) Veröffentlichungstag der Patentschrift: **08.01.92**

(51) Int. Cl.5: **C07D 209/08**

(21) Anmeldenummer: **86810498.5**

(22) Anmeldetag: **03.11.86**

Die Akte enthält technische Angaben, die nach dem Eingang der Anmeldung eingereicht wurden und die nicht in dieser Patentschrift enthalten sind.

(54) **Verfahren zur Herstellung von Indolinen.**

(30) Priorität: **08.11.85 US 796358**
**11.07.86 US 884836**

(43) Veröffentlichungstag der Anmeldung:
**13.05.87 Patentblatt 87/20**

(45) Bekanntmachung des Hinweises auf die Patenterteilung:
**08.01.92 Patentblatt 92/02**

(84) Benannte Vertragsstaaten:
**AT BE CH DE ES FR GB GR IT LI LU NL SE**

(56) Entgegenhaltungen:
**DE-C- 606 027**

**ACTA CHEMICA SCANDINAVICA, Band B 28, Teil 1, Serie B, 1974, Kopenhagen, Dänemark; J. BAKKE et al. "A New One Step Indole Synthesis" Seiten 393-398**

(73) Patentinhaber: **CIBA-GEIGY AG**
**Klybeckstrasse 141**
**CH-4002 Basel(CH)**

(72) Erfinder: **Aebli, Beat M.**
**Lothringerstrasse 1**
**CH-4004 Basel(CH)**
Erfinder: **Gremmelmaier, Claude, Dr.**
**Eichbergweg 6**
**CH-4147 Aesch(CH)**

**EP 0 221 854 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Indolinen der Formel

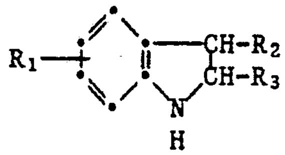 (I)

in welcher $R_1$ Wasserstoff oder $C_1$-$C_4$-Alkyl bedeutet und $R_2$ und $R_3$ unabhängig voneinander je für Wasserstoff, $C_1$-$C_4$-Alkyl oder Phenyl stehen, durch Cyclodehydratisierung eines 2-(2'-Aminophenyl)-äthanols der Formel II

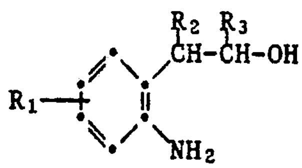 (II)

in welcher $R_1$, $R_2$ und $R_3$ die unter Formel I angegebene Bedeutung haben, in Gegenwart eines Katalysators.

Die Indoline der Formel I sind Zwischenprodukte zur Herstellung von Pyrrolo[3,2,1-i,j]chinolinen mit bakteriziden und fungiziden Eigenschaften, welche zur Bekämpfung von Pflanzenkrankheiten verwendet werden können. Solche Pyrrolo[3,2,1-i,j]chinoline sind beispielsweise in den Britischen Patentschriften 1 394 373 und 1 394 374 beschrieben. Als Vertreter dieser Stoffklasse sei insbesondere das 4-Lilolidon (1,2,5,6-Tetrahydro-4H-pyrrolo[3,2,1-i,j]chinolin-2-on) der Formel III

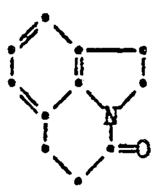 (III)

erwähnt, dass auch unter der Bezeichnung Pyroquilon bekannt geworden ist.

Aus der Deutschen Patentschrift 606 027 ist bekannt, Indolin in der Gasphase herzustellen, indem man 2-(2'-Aminophenyl)-äthanol in Gegenwart eines Kondensationsmittels auf 150-500 °C, insbesondere 200-400 °C erhitzt. Als Beispiele für Kondensationsmittel werden die Oxide von Aluminium, Titan, Zirkon, Chrom, Thorium oder Gemische dieser Oxide erwähnt. Ferner können nach den Angaben der vorgenannten Patentschrift auch andere Verbindungen, wie Sulfate, Phosphate, Silikate, Hydrosilikate, Borate, Chloride, Mineralsäuren und Säureanhydride entweder an Stelle der vorgenannten Oxide oder im Gemisch mit ihnen als Kondensationsmittel verwendet werden.

Ferner ist es aus Acta Chem. Scand. B 28 (1974), 393-398 bekannt, Indolin durch Ueberleiten von 2-(2'-Aminophenyl)-äthanol-Dampf und Wasserstoff über Silicagel bei 250 °C herzustellen. Die mit diesem Verfahren erzielbare Ausbeute wird mit 98 % angegeben. Der wesentliche Nachteil dieses Verfahrens besteht darin, dass Silicagel bereits nach kurzer Zeit seine katalytische Aktivität verliert. Das Verfahren ist daher für eine technische Herstellung von Indolin wenig geeignet.

Es ist ferner bekannt, Indoline durch Cyclodehydratisierung entsprechender 2-(2'-Aminophenyl)-äthanole in flüssiger Phase herzustellen. So ist in der publizierten Japanischen Patentanmeldung Sho 52-108.969 ein Verfahren beschrieben, nach welchem Indoline durch blosses Erhitzen von 2-(2'-Aminophenyl)-äthanol auf 200-300 °C in einer Apparatur aus Pyrex-Glas hergestellt werden, wobei der bei der Umsetzung herrschende Druck so gewählt wird, dass das eingesetzte 2-(2'-Aminophenyl)-äthanol zumindest zum Teil flüssig bleibt. Für dieses Verfahren wird bei einer Reaktionstemperatur von 250 °C und einer Reaktionszeit von 2 Stunden ausgehend von 2-(2'-Aminophenyl)-äthanol eine Ausbeute an Indolin von 99 % der Theorie und bei einer Reaktionstemperatur von 260 °C und einer Reaktionszeit von 6 Stunden ausgehend von

substituierten 2-(2'-Aminophenyl)-äthanol eine Ausbeute von 77-94 % der Theorie an entsprechend substituierten Indolinen beschrieben. Diese Angaben über die erzielbaren Ausbeuten konnten jedoch bei der Nacharbeitung des Verfahrens nicht bestätigt werden. So wurde bei analoger Durchführung des Verfahrens ausgehend von 2-(2'-Aminophenyl)-äthanol bei einer Reaktionszeit von 2,75 Stunden und einer Reaktionstemperatur von 250 °C lediglich ein Umsatz von 2,4 % erzielt, was bei einer Selektivität von 42 % einer Ausbeute von 1 % der Theorie entspricht. Bei gleicher Reaktionszeit von 2,75 Stunden und einer Temperatur von 280 °C betrug der Umsatz 5,5 %, die Selektivität 38 % und die Ausbeute 2,1 % der Theorie. Bei einer Reaktionszeit von 7 Stunden und einer Reaktionstemperatur von 300 °C lag der Umsatz bei 18,3 %, die Selektivität bei 83 % und die Ausbeute bei 15,3 % der Theorie. Bei Verwendung von speziell gereinigtem 2-(2'-Aminophenyl)-äthanol (99,9%ig) und Durchführung der Reaktion in einer gereinigten Glasapparatur wurde bei einer Reaktionszeit von 2 Stunden und einer Reaktionstemperatur von 250 °C lediglich ein Umsatz von 0,5 % erreicht.

Aus der Japanischen Patentanmeldung SHO 58-146.562 ist ein Verfahren bekannt, nach dem Indolin in der Weise hergestellt wird, dass man 2-(2'-Aminophenyl)-äthanol-Dampf bei 180-300 °C über einen Katalysator leitet, der aus mit einer Bor- oder Phosphorverbindung imprägnierten Tonerde oder Kieselgel besteht. Wobei als Bor- oder Phosphorverbindung in erster Linie Borsäure und Phosphorsäure in Betracht kommen. Das Verfahren ist insofern nachteilig als der verwendete Katalysator seine Aktivität rasch verliert. Ausserdem muss das Verfahren bei vermindertem Druck durchgeführt werden, was einen verhältnismässig hohen apparativen Aufwand bedingt.

In der publizierten Japanischen Patentanmeldung 58-146.563 wird ein Verfahren beschrieben, nach dem die Cyclodehydratisierung von 2-(2'-Aminophenyl)-äthanol zu Indolin in flüssiger Phase bei Temperaturen von 200-220 °C in Gegenwart von Phosphorsäure, Borsäureanhydrid, Schwefelsäure oder Salpetersäure auf $\gamma$-Aluminiumoxid als Träger oder von Phosphorsäure, Borsäure oder Borsäureanhydrid auf Titandioxid als Träger durchgeführt wird. Nach diesem Verfahren wird mit einem aus $\gamma$-Aluminiumoxid und Schwefelsäure bestehenden Katalysator, der in einer Menge von 10 Gew.% bezogen auf eingesetztes 2-(2'-Aminophenyl)-äthanol verwendet wird, bei einer Reaktionszeit von 1,5 Stunden und einer Reaktionstemperatur von 216-218 °C Indolin in einer Ausbeute von 96,3 % der Theorie und einer Reinheit von 97,7 % erhalten. Ein wesentlicher Nachteil dieses Verfahrens besteht darin, dass zur Erreichung einer zufriedenstellenden Produktivität hohe Katalysatormengen erforderlich sind.

Nach einem in der publizierten Britischen Patentanmeldung 2 501 065 A beschriebenen Verfahren werden Indoline hergestellt, indem man entsprechende 2-(2'-Aminophenyl)-äthanol-hydrohalogenide in flüssiger Phase auf Temperaturen über 120 °C erhitzt, wobei die flüssige Phase durch die Schmelze des eingesetzten Hydrohalogenids oder eine wässrige Lösung desselben gebildet werden kann. Ein wesentlicher Nachteil dieses Verfahrens besteht darin, dass die gewünschten Indoline nicht in freier From sondern in Form ihrer Hydrohalogenide erhalten werden, aus welchem sie in einer weiteren Verfahrensstufe freigesetzt und isoliert werden müssen.

Es ist das Ziel der vorliegenden Erfindung, die Nachteile der bekannten Verfahren zu vermeiden und ein Verfahren bereitzustellen, nach dem die Indoline der Formel I direkt durch Cyclodehydratisierung von 2-(2'-Aminophenyl)-äthanol der Formel II in guter Ausbeute erhalten werden können.

Es wurde gefunden, dass man die Indoline der Formel I in ausgezeichneter Ausbeute durch Cyclodehydratisierung eines 2-(2'-Aminophenyl)-äthanols der Formel II herstellen kann, wenn man die Cyclodehydratisierung des 2-(2'-Aminophenyl)-äthanols der Formel II bei 150-350 °C in Gegenwart eines amorphen Alumosilikats (Silica Alumina) durchführt.

Das erfindungsgemässe Verfahren kann sowohl in der Gasphase als auch in flüssiger Phase durchgeführt werden.

Als geeignete amorphe Alumosilikate, in deren Gegenwart das erfindungsgemässe Verfahren durchgeführt wird, sind insbesondere solche mit 5-30 Gew.% $Al_2O_3$ und 70-95 Gew.% $SiO_2$, einer inneren Oberfläche gemessen nach BET von 50-800 $m^2/g$ und einem Porenvolumen von 0,3-1,0 $cm^3/g$ geeignet. Vorzugsweise werden amorphe Alumosilikate mit 10-20 Gew.% $al_2O_3$ und 80-90 Gew.% $SiO_2$, einer inneren Oberfläche gemessen nach BET von 300-600 $m^2/g$ und einem Porenvolumen von 0,4-0.7 $cm^3/g$ verwendet. Besonders bevorzugt sind amorphe Alumosilikate, die 10-15 Gew.% $Al_2O_3$ und 85-90 Gew.% $SiO_2$ enthalten, mit einer inneren Oberfläche gemessen nach BET von 300-500 $m^2/g$ und einem Porenvolumen von 0,5-0,7 $cm^3/g$. Die Angaben über den Gehalt der erfindungsgemäss zu verwendenden amorphen Alumosilikate an Aluminiumoxid ($Al_2O_3$) und Siliciumdioxid ($SiO_2$) beziehen sich jeweils auf die als feste Oxide bestimmbaren Mengen dieser Komponenten.

Die erfindungsgemäss als Katalysatoren zu verwendenden amorphen Alumosilikate können allgemein durch Zugabe einer wässrigen Lösung einer Aluminiumsalzes, beispielsweise Aluminiumsulfat, zu einer wässrigen Lösung einer Silikats, beispielsweise Natriumsilikat, Filtration des gebildeten Niederschlages,

Auswaschen mit Wasser und Calcinieren hergestellt werden. Die Herstellung der Katalysatoren kann auch in der Weise erfolgen, dass man aus einer wässrigen Lösung von Natriumsilikat durch Zugabe von Schwefelsäure Kieselgel Ausfällt und diesem nach einer gewissen Alterungszeit Aluminiumsulfat und Ammoniak zusetzt, wobei der pH-Wert im schwach sauren Bereich gehalten wird. Danach wird der Niederschlag abfiltriert ausgewaschen, getrocknet und calciniert. Die Herstellung von erfindungsgemäss verwendbaren Katalysatoren ist beispielsweise in den US-Patentschriften 2 283 172 und 2 283 173 beschrieben. Bezüglich weiterer Angaben über die Struktur solcher erfindungsgemäss verwendbarer amorpher Alumosilikate sei auch auf Ind.Eng.Chem. 41, (1949), 2564-73 verwiesen.

Erfindungsgemäss als Katalysatoren verwendbare amorphe Alumosilikate sind auch im Handel erhältlich, beispielsweise unter den Bezeichnungen ®KETJEN LA-100-3P und ®KETJEN C-25-W (Akzo Chemie, Amsterdam), sowie HOUDRY-HUELS S-90 (Chemische Werke Hüls AG).

Nach einer bevorzugten Ausführungsform des erfindungsgemässen Verfahrens werden die Indoline der Formel I in ausgezeichneter Ausbeute erhalten, wenn man die Cyclodehydratisierung des 2-(2'-Aminophenyl)-äthanols der Formel II in der Gasphase bei 200-300 °C in Gegenwart eines amorphen Alumosilikats (Silica Alumina) mit 5-30 Gew.% $Al_2O_3$ und 70-95 Gew.% $SiO_2$, einer inneren Oberfläche gemessen nach BET von 50-800 $m^2/g$ und einem Porenvolumen von 0,1-1,0 $cm^3/g$ durchführt.

Innerhalb des angegebenen Bereichs von 200-300 °C sind Reaktionstemperaturen von 240-260 °C bevorzugt.

Das erfindungsgemässe Verfahren wird in der Gasphase vorteilhaft in der Weise durchgeführt, dass man pro Kilogramm Katalysator und Stunde 0,1-10 kg 2-(2'-Aminophenyl)-äthanol der Formel II durchsetzt. Vorsugsweise werden pro Kilogramm Katalysator und Stunde 0,5-2 kg 2-(2'-Aminophenyl)-äthanol der Formel II durchgesetzt.

Es ist ferner vorteilhaft, das erfindungsgemässe Verfahren in der Gasphase in Gegenwart eines Trägergases durchzuführen. Geeignete Trägergase sind beispielsweise Stickstoff, Wasserstoff und Wasserdampf. Vorzugsweise wird das erfindungsgemässe Verfahren in Gegenwart von Wasserdampf als Trägergas durchgeführt.

Das Trägergas wird in der Regel in einer Menge von 1-10 Mol pro Mol 2-(2'-Aminophenyl)-äthanol der Formel II eingesetzt. Vorzugsweise verwendet man 6-8 Mol Trägergas pro Mol 2-(2'-Aminophenyl)-äthanol der Formel II.

Die Mischungen von Trägergas und dem Dampf des 2-(2'-Aminophenyl)-äthanol der Formel II werden zweckmässig in der Weise hergestellt, dass man dem 2-(2'-Aminophenyl)-äthanol-Dampf das jeweilige Trägergas zudosiert. Bei der Verwendung von Wasserdampf als Trägergas kann man in vorteilhafter Weise so vorgehen, dass man eine wässrige Lösung eines 2-(2'-Aminophenyl)-äthanols der Formel II verdampft. Hierzu sind insbesondere wässrige Lösungen geeignet, die 40-60 Gew.% 2-(2'-Aminophenyl)-äthanol der Formel II enthalten.

Die erfindungsgemäss vorgeschlagenen Katalysatoren können in einem Festbett, beispielsweise in einem Röhrenreaktor, oder in einem Fliessbett, beispielsweise in einem Wirbelschichtreaktor, angewendet werden. In einem Festbett werden die erfindungsgemäss vorgeschlagenen Katalysatoren vorteilhaft in pelletierter Form verwendet.

Der Druck, bei dem das erfindungsgemässe Verfahren in der Gasphase durchgeführt wird, ist nicht kritisch. Vorzugsweise wird das Verfahren bei Normaldruck durchgeführt. Das Verfahren kann jedoch ebenfalls bei Unter- oder Ueberdruck durchgeführt werden, wobei jedoch durch geeignete Wahl der Reaktionstemperatur und durch Verwendung von Trägergas dafür Sorge zu tragen ist, dass das Reaktionsgemisch gasförmig vorliegt.

Nach einer weiteren bevorzugten Ausführungsform des erfindungsgemässen Verfahrens werden die Indoline der Formel I in ausgezeichneter Ausbeute erhalten, wenn man die Cyclodehydratisierung des 2-(2'-Aminophenyl)-äthanols der Formel II bei 150-350 °C in Gegenwart eines amorphen Alumosilikats (Silica Alumina) mit 5-30 Gew.% $Al_2O_3$ und 70-95 Gew.% $SiO_2$, einer inneren Oberfläche gemessen nach BET von 50-800 $m^2/g$ und einem Porenvolumen von 0,1-1,0 $cm^3/g$ in flüssiger Phase durchführt.

Innerhalb des angegebenen Bereichs von 150-350 °C sind Reaktionstemperaturen von 200-280 °C bevorzugt.

Die Durchführung des erfindungsgemässen Verfahrens in der Flüssigen Phase kann diskontinuierlich in einzelnen Ansätzen oder kontinuierlich erfolgen. Vorzugsweise wird das erfindungsgemässe Verfahren kontinuierlich durchgeführt.

Bei der diskontinuierlichen Arbeitsweise geht man zweckmässig so vor, dass man das amorphe Alumosilikat in dem als Ausgangsmaterial verwendeten 2-(2'-Aminophenyl)-äthanol der Formel II suspendiert und die Suspension auf 150-350 °C, vorzugsweise 200-280 °C, erhitzt, wobei der Druck so gewählt wird, dass er über dem von Reaktionsgemisch bei Reaktionstemperatur entwickelten Dampfdruck liegt.

Dabei dient als Reaktionsgefäss vorzugsweise ein mit einem Rührer versehener Autoklav. Die Reaktionszeit beträgt je nach Reaktionstemperatur und Katalysatormenge 0,5-3 Stunden. Nach beendigter Umsetzung wird das Reaktionsgemisch durch fraktionierte Destillation aufgearbeitet. Da die Umsetzung zum Indolin der Formel I in den meisten Fällen praktisch quantitativ verläuft, genügt es in der Regel, das bei der Reaktion gebildete Wasser abzudestillieren und den Katalysator abzutrennen, um zu einem für die meisten Zwecke brauchbaren Produkt zu gelangen. Das nach dem Abtrennen des Reaktionswassers und des Katalysators erhaltene Produkt kann jedoch gewünschtenfalls auch durch Destillation gereinigt werden, um die bei der Reaktion stets in geringer Menge gebildeten hochsiedenden Nebenprodukte abzutrennen. Gemäss einer vorteilhaften Variante wird das Reaktionswasser bereits während der Umsetzung abdestilliert. In diesem Fall wird bei einem Druck gearbeitet, der über dem bei der jeweiligen Reaktionstemperatur von Ausgangs- und Endprodukt entwickelten Dampfdruck liegt. Man gelangt so nach beendigter Umsetzung und Abtrennung des amorphen Alumosilikats direkt zu den gewünschten Indolinen.

Bei der kontinuierlichen Durchführung des Verfahrens geht man zweckmässig so vor, dass man das 2-(2'-Aminophenyl)-äthanol der Formel II und das amorphe Alumosilikat in einem mit einem Rührer, einer Dosiervorrichtung und einer Destillationskolonne versehenen Reaktionsgefäss bei einem dem Dampfdruck des Reaktionsgemisches bei der jeweiligen Reaktionstemperatur entsprechenden Druck auf Reaktionstemperatur erhitzt, die gebildeten Reaktionsprodukte Wasser und Indolin der Formel I über die Kolonne abdestilliert und dem Reaktionsgemisch über die Dosiervorrichtung ständig eine der abdestillierten Menge Wasser und Indolin entsprechende Menge frisches 2-(2'-Aminophenyl)-äthanol der Formel II zuführt. Dabei wird die Destillationsgeschwindigkeit und die dieser entsprechende Dosiergeschwindigkeit vorteilhaft so gewählt, dass die mittlere Verweilzeit im Reaktionsgefäss je nach der gewählten Reaktionstemperatur und der eingesetzten Katalysatormenge 0,5-3 Stunden beträgt.

Da bei der Cyclodehydratisierung von 2-(2'-Aminophenyl)-äthanol der Formel II in flüssiger Phase unter den erfindungsgemässen Bedingungen stets geringe Mengen an hochsiedenden Nebenprodukten gebildet werden, erhöht sich mit fortschreitender Dauer bei der kontinuierlichen Durchführung des Verfahrens der Siedepunkt des Reaktionsgemisches. Um trotzdem eine konstante Produktionsgeschwindigkeit aufrechterhalten zu können, ist es daher notwendig, entweder die Reaktionstemperatur ständig entsprechend zu erhöhen oder den Druck ständig entsprechend zu verringern. Wenn der Anteil der hochsiedenden Nebenprodukte im Reaktionsgemisch zu hoch geworden ist, müssen diese abgetrennt werden. Dies kann in einfacher Weise so geschehen, dass man die Zudosierung des 2-(2'-Aminophenyl)-äthanols der Formel II unterbricht, das im Reaktionsgemisch vorhandene Indolin der Formel I aus dem Reaktionsgefäss herausdestilliert, aus dem Destillationsrückstand die hochsiedenden Nebenprodukte durch Filtration vom amorphen Alumosilikat abtrennt und mit dem gleichen oder mit frischem amorphen Alumosilikat einen neuen Zyklus beginnt.

Die Menge an amorphen Alumosilikat beträgt bei diskontinuierlicher Arbeitsweise in flüssiger Phase in der Regel 0,5-10 Gew.% des eingesetzten 2-(2'-Aminophenyl)-äthanols und bei kontinuierlicher Arbeitsweise 0,5-10 Gew.% der Menge des im Reaktionsgefäss vorhandenen Reaktionsgemisches. Vorzugsweise wird das amorphe Alumosilikat bei diskontinuierlicher Arbeitsweise in einer Menge von 1-5 Gew.% des eingesetzten 2-(2'-Aminophenyl)-äthanols der Formel II und bei kontinuierlicher Arbeitsweise in einer Menge von 1-5 Gew.% des im Reaktionsgefäss vorhandenen Reaktionsgemisches verwendet.

Die als Ausgangsmaterialien zu verwendenden 2-(2'-Aminophenyl)-äthanole der Formel II können in an sich bekannter Weise durch Umsetzung entsprechender 2-Nitroalkylbenzole mit aliphatischen und aromatischen Aldehyden und anschliessende Reduktion der erhaltenen 2-(2'Nitrophenyl)-äthanole hergestellt werden. Die Umsetzung von 1-Nitroalkylbenzolen mit aliphatischen oder aromatischen Aldehyden ist beispielsweise in der publizierten japanischen Patentanmeldung 77.108.941 beschreiben. Die Reduktion der 2-(2'-Nitrophenyl)-äthanole kann nach Bull. Soc. Chim, France, (1931), 49, Seite 3, mit Zink durchgeführt werden.

Das erfindungsgemässe Verfahren eignet sich insbesondere zur Herstellung von Indolin. Dementsprechend stellt 2-(2'Aminophenyl)-äthanol das bevorzugte Ausgangsmaterial dar. Gemäss einer bevorzugten Variante des erfindungsgemässen Verfahrens wird Indolin hergestellt, indem man 2-(2'-Aminophenyl)-äthanol bei 240-260 °C in der Gasphase in Gegenwart eines amorphen Alumosilikats mit 10-15 Gew.% $Al_2O_3$ und 85-90 Gew.% $SiO_2$, einer inneren Oberfläche gemessen nach BET von 300-500 $m^2/g$ und einem Porenvolumen von 0,5-0,7 $cm^3/g$ als Katalysator und in Gegenwart von 6-8 Mol Wasserdampf pro Mol 2-(2'-Aminophenyl)-äthanol bei einem Durchsatz von 0,5-2 kg 2-(2'-Aminophenyl)-äthanol pro Kilogramm Katalysator und Stunde cyclodehydratisiert.

Gemäss einer weiteren bevorzugten Variante des erfindungsgemässen Verfahrens wird Indolin hergestellt, indem man 1-(2'-Aminophenyl)-äthanol in flüssiger Phase in Gegenwart von 1-5 Gew.% eines amorphen Alumosilikats mit 10-15 Gew.% $Al_2O_3$, 85-90 Gew.% $SiO_2$, einer inneren Oberfläche gemessen nach BET von 300-500 $m^2/g$ und einem Porenvolumen von 0,5-0,7 $cm^3/g$, bezogen auf die Menge des im

Reaktor vorhandenen Reaktionsgemisches, bei einer mittleren Verweilzeit von 0,5-3 Stunden cyclodehydratisiert.

Mit dem erfindungsgemässen Verfahren können Umsätze von 2-(2'-Aminophenyl)-äthanol der Formel II von 98-100 % und in Bezug auf die Bildung von Indolinen der Formel I Selektivitäten von bis zu 98-99 % erreicht werden. Der besondere Vorteil des erfindungsgemässen Verfahrens gegenüber den bisher bekannten Verfahren besteht darin, dass die erfindungsgemäss als Katalysatoren zu verwendenden amorphen Alumosilikate ihre Aktivität wesentlich länger beibehalten, als die bisher für die Cyclodehydratisierug von 2-(2'-Aminophenyl)-äthanol verwendeten Katalysatoren, wobei sich das in flüssiger Phase durchgeführte Verfahren gegenüber des Gasphasenverfahren durch eine bessere Volumenausbeute auszeichnet.

Das erfindungsgemässe Verfahren wird durch die folgenden Beispiele näher erläutert:

Beispiel 1:

In einem Röhrenreaktor aus Pyrexglas mit einem inneren Durchmesser von 28 mm werden 8,3 g/h 2-(2'-Aminophenyl)-äthanol zusammen mit 8,3 g/h Wasserdampf bei 250° C über 15,0 g eines pelletierten amorphen Aluminiumsilikats mit 13.5 Gew.% $Al_2O_3$ und 86 Gew.% $SiO_2$, einer inneren Oberfläche gemessen nach BET von 418 $m^2/g$ und einem Porenvolumen von 0,51 $cm^3/g$ (®KETJEN LA-100-3P; Akzo Chemie, Amsterdam) geleitet. Bei einer Versuchsdauer von 1500 Stunden wird bei einem Umsatz von 98-100 % bezogen auf eingesetztes 2-(2'-Aminophenyl)-äthanol Indolin mit einer Selektivität von 98-99 % gebildet. Der Umsatz beträgt bei Versuchsende unverändert 98-100 % bezogen auf eingesetztes 2-(2'-Aminophenyl)-äthanol.

Beispiel 2:

In einem Röhrenreaktor aus Pyrexglas mit einem inneren Durchmesser von 28 mm werden 18,9 g/h durch Verdampfen einer 50 %-igen wässrigen Lösung von 2-(2'-Aminophenyl)-äthanol gewonnenes Gasgemisch bei 250° C über 8,6 g eines amorphen Alumosilikats mit 12,4 Gew.% $Al_2O_3$ und 87,3 Gew.% $SiO_2$, einer inneren Oberfläche gemessen nach BET von 317 $m^2/g$ und einem Porenvolumen von 0.64 $cm^3/g$ (Houdry-Hüls S-90 geleitet. Dabei wird bei einer Versuchsdauer von 730 Stunden ohne zwischenzeitliche Regeneration des Katalysators bei einem Umsatz von 99-100 % bezogen auf eingesetztes 2-(2'-Aminophenyl)-äthanol mit einer Selektivität von 98-99 % Indolin gebildet. Bei Versuchsende ist noch keine Abnahme des Umsatzes festzustellen.

Beispiel 3:

In einem mit einer Destillationskolonne versehenen Reaktor aus rostfreiem Stahl werden 5,0 g eines handelsüblichen amorphen Alumosilikats (®KETJEN C-25-W) mit einem $Al_2O_3$-Gehalt von 14,7 Gew.%, einem $SiO_2$-Gehalt von 82,5 Gew.%, eine inneren Oberfläche gemessen nach BET von 547 $m^2/g$ und einem Porenvolumen von 0,61 $cm^3/g$ mit Hilfe eines Magnetrührers in 130 g (117 ml) 2-(2'-Aminophenyl)-äthanol suspendiert. Die Suspension wird auf 220° C erhitzt. Dann wird bei einer konstanten Reaktionstemperatur von 220° C und einem anfänglichen Druck von 0,5 bar das entstandene Indolin-Wasser-Gemisch mit einer mittleren Geschwindigkeit von 60 g Indolin pro Stunde über die Füllkörperkolonne abdestilliert. Dabei wird das Volumen im Reaktor mittels einer Niveausonde durch kontinuierliches Zudosieren von frischem 2-(2'-Aminophenyl)-äthanol konstant gehalten. Um die durch Bildung hochsiedender Nebenprodukte verursachte Herabsetzung der Destillationsgeschwindigkeit auszugleichen, wird der Druck zur Erhaltung der Destillationsgeschwindigkeit während der Versuchsdauer allmählich von 0,5 bar auf 0,07 bar abgesenkt. Nach einer Versuchsdauer von 150 Stunden wird die Zudosierung von 2-(2'-Aminophenyl)-äthanol unterbrochen und das im Reaktor befindliche Indolin unter vermindertem Druck herausdestilliert. Es werden so 9,0 kg Indolin mit einer Reinheit von 99,5 % als Destillat erhalten. Als Destillationsrückstand werden 55 g hochsiedende Nebenprodukte unbekannter Konstitution erhalten. Die Ausbeute an Indolin beträgt 98-99 % der Theorie.

Beispiel 4:

In einem mit einer Destillationskolonne versehenen Reaktor aus rostfreiem Stahl werden 0,8 g eines handelsüblichen amorphen Alumosilikats (®KETJEN LA-100-3P) mit einem $Al_2O_3$-Gehalt von 13,3 Gew.%, einem $SiO_2$-Gehalt von 86,3 Gew.% einer inneren Oberfläche gemessen nach BET von 403 $m^2/g$ und einem Porenvolumen von 0.52 $cm^3/g$ mit Hilfe eines Magnetrührers in 80 g (72 ml) 2-(2'-Aminophenyl)-äthanol suspendiert. Die Suspension wird unter Normaldruck auf 240° erhitzt und das gebildete Indolin-Wasser-Gemisch mit einer Geschwindigkeit von 25 g Indolin pro Stunde abdestilliert, wobei das Volumen im Reaktionsgefäss mit einer Niveausonde durch kontinuierliches Zudosieren von 2-(2'-Aminophenyl)-äthanol konstant gehalten wird. Zur Aufrechterhaltung dieser Destillationsgeschwindigkeit wird die Reaktionstemperatur während der gesamten Versuchsdauer von 126 Stunden von anfangs 240 °C allmählich auf 270 °C erhöht. Nach 126 Stunden wird die Zudosierung von 2-(2'-Aminophenyl)-äthanol unterbrochen und das restliche im Reaktor befindliche Indolin über die Kolonne abdestilliert. Es werden so 2,95 kg (97-98 % der Theorie) Indolin mit einer Reinheit von 99 % erhalten.

## Patentansprüche

1. Verfahren zur Herstellung von Indolinen der Formel I

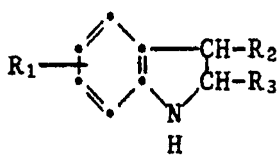

(I)

in welcher $R_1$ Wasserstoff oder $C_1$-$C_4$-Alkyl bedeutet und $R_2$ und $R_3$ unabhängig voneinander je für Wasserstoff, $C_1$-$C_4$-Alkyl oder Phenyl stehen, durch Cyclodehydratisierung eines 2-(2'-Aminophenyl)-äthanols der Formel II

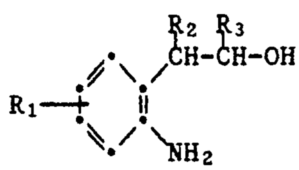

(II)

in welcher $R_1$, $R_2$ und $R_3$ die unter Formel I angegebene Bedeutung haben, in Gegenwart eines Katalysators, dadurch gekennzeichnet, dass man die Cyclodehydratisierung des 2-(2'-Aminophenyl)-äthanols der Formel II bei 150-350 °C in Gegenwart eines amorphen Alumosilikats (Silica Alumina) durchführt.

2. Verfahren nach Anspruch 1, dadurch gekennzeichnet, dass man die Cyclodehydratisierung des 2-(2'-Aminophenyl)-äthanols der Formel II in der Gasphase bei 200-300 °C in Gegenwart eines amorphen Alumosilikats (Silica Alumina) mit 5-30 Gew.% $Al_2O_3$ und 70-95 Gew.% $SiO_2$, einer inneren Oberfläche gemessen nach BET von 50-800 $m^2/g$ und einem Porenvolumen von 0,1-1,0 $cm^3/g$ durchführt.

3. Verfahren nach Anspruch 2, dadurch gekennzeichnet, dass man die Cyclodehydratisierung des 2-(2'-Aminophenyl)-äthanols der Formel II bei 240-260 °C durchführt.

4. Verfahren nach Anspruch 2, dadurch gekennzeichnet, dass man als Katalysator ein amorphes alumosilikat mit 10-20 Gew.% $Al_2O_3$ und 80-90 Gew.% $SiO_2$, einer inneren Oberfläche gemessen nach BET von 300-600 $m^2/g$ und einem Porenvolumen von 0,4-0,7 $cm^3/g$ verwendet.

5. Verfahren nach Anspruch 4, dadurch gekennzeichnet, dass man als Katalysator ein amorphes Alumosilikat mit 10-15 Gew.% $Al_2O_3$ und 85-90 Gew.% $SiO_2$, einer inneren Oberfläche gemessen nach BET von 300-500 $m^2/g$ und einem Porenvolumen von 0,5-0,7 $cm^3/g$ verwendet.

6. Verfahren nach Anspruch 2, dadurch gekennzeichnet, dass man pro Kilogramm Katalysator und Stunde 0,1-10 kg 2-(2'-Aminophenyl)-äthanol der Formel II durchsetzt.

7. Verfahren nach Anspruch 6, dadurch gekennzeichnet, dass man pro Kilogramm Katalysator und Stunde 0,5-2 kg 2-(2'-Aminophenyl)-äthanol der Formel II durchsetzt.

8. Verfahren nach Anspruch 2, dadurch gekennzeichnet, dass man die Cyclodehydratisierung des 2-(2'-Aminophenyl)-äthanols der Formel II in Gegenwart eines Trägergases durchführt.

9. Verfahren nach Anspruch 8, dadurch gekennzeichnet, dass man als Trägergas Stickstoff, Wasserstoff oder Wasserdampf verwendet.

10. Verfahren nach Anspruch 8, dadurch gekennzeichnet, dass man als Trägergas Wasserdampf verwendet.

11. Verfahren nach Anspruch 8, dadurch gekennzeichnet, das man 1-10 Mol Trägergas pro Mol 2-(2'-Aminophenyl)-äthanol der Formel II verwendet.

12. Verfahren nach Anspruch 8, dadurch gekennzeichnet, dass man 6-8 Mol Trägergas pro Mol 2-(2'-Aminophenyl)-äthanol der Formel II verwendet.

13. Verfahren nach Anspruch 2, dadurch gekennzeichnet, dass man 2-(2'-Aminophenyl)-äthanol cyclodehydratisiert.

14. Verfahren nach Anspruch 13, dadurch gekennzeichnet, dass man 2-(2'-Aminophenyl)-äthanol bei 240-260 °C in Gegenwart eines amorphen Alumosilikats mit 10-15 Gew.% $Al_2O_3$ und 85-90 Gew.%, $SiO_2$, einer inneren Oberfläche gemessen nach BET von 300-500 $m^2/g$ und einem Porenvolumen von 0,5-0,7 $cm^3/g$ als Katalysator und in Gegenwart von 6-8 Mol Wasserdampf pro Mol 2-(2'-Aminophenyl)äthanol bei einem Durchsatz von 0,5-2 kg 2-(2'-Aminophenyl)-äthanol pro Kilogramm Katalysator und Stunde cyclodehydratisiert.

15. Verfahren nach Anspruch 1, dadurch gekennzeichnet, dass man die Cyclodehydratisierung des 2-(2'-Aminophenyl)-äthanols der Formel II in flüssiger Phase bei 150-350 °C in Gegenwart eines amorphen Alumosilikats mit 5-30 Gew.% $Al_2O_3$ und 70-95 Gew.% $SiO_2$, einer inneren Oberfläche gemessen nach BET von 50-800 $m^2/g$ und einem Porenvolumen von 0,1-1,0 $cm^3/g$ durchführt.

16. Verfahren nach Anspruch 15, dadurch gekennzeichnet, dass man die Cyclodehydratisierung des 2-(2'-Aminophenyl)-äthanols der Formel II in Gegenwart eines amorphen Alumosilikats mit 10-20 Gew.% $Al_2O_3$ und 80-90 Gew.% $SiO_2$, einer inneren Oberfläche gemessen nach BET von 300-600 $m^2/g$ und einem Porenvolumen von 0,4-0,7 $cm^3/g$ durchführt.

17. Verfahren nach Anspruch 16, dadurch gekennzeichnet, dass man die Cyclodehydratisierung des 2-(2'-Aminophenyl)-äthanols der Formel II in Gegenwart eines amoprphen Alumosilikats mit 10-15 Gew.% $Al_2O_3$ und 85-90 Gew.% $SiO_2$, einer inneren Oberfläche gemessen nach BET von 300-500 $m^2/g$ und einem Porenvolumen von 0,5-0,7 $cm^3/g$ durchführt.

18. Verfahren nach Anspruch 15, dadurch gekennzeichnet, dass man die Cyclodehydratisierung des 2-(2'-Aminophenyl)-äthanols der Formel II bei einer Temperatur 200-280 °C durchführt.

19. Verfahren nach Anspruch 15, dadurch gekennzeichnet, dass man es kontinuierlich oder diskontinuierlich durchführt.

20. Verfahren nach Anspruch 19, dadurch gekennzeichnet, dass man es kontinuierlich durchführt.

21. Verfahren nach Anspruch 15, dadurch gekennzeichnet, dass man es diskontinuierlich durchführt und das amorphe Alumosilikat in einer Menge von 0,5-10 Gew.% des eingesetzten 2-(2'-Aminophenyl)-äthanols der Formel II verwendet.

22. Verfahren nach Anspruch 15, dadurch gekennzeichnet, dass man es kontinuierlich durchführt und das amorphe Alumosilikat in einer Menge von 0,5-10 Gew.% der Menge des im Reaktionsgefäss vorhandenen Reaktionsgemisches verwendet.

23. Verfahren nach Anspruch 22, dadurch gekennzeichnet, dass man das amorphe Alumosilikat in einer Menge von 1-5 Gew.% des im Reaktionsgefäss vorhandenen Reaktionsgemisches verwendet.

24. Verfahren nach Anspruch 15, dadurch gekennzeichnet, dass man 2-(2'-Aminophenyl)-äthanol cyclodehydratisiert.

25. Verfahren nach Anspruch 24, dadurch gekennzeichnet, dass man 2-(2'-Aminophenyl)-äthanol in Gegenwart von 1-5 Gew.% eines amorphen Alumosilikats mit 10-15 Gew.% $Al_2O_3$, 85-90 Gew.% $SiO_2$, einer inneren Oberfläche gemessen nach BET von 300-500 $m^2/g$ und einem Porenvolumen von 0,5-0,7 $cm^3/g$, bezogen auf die Menge des im Reaktor vorhandenen Reaktionsgemisches, bei einer mittleren Verweilzeit von 0,5-3 Stunden cyclodehydratisiert.

## Claims

1. A process for the preparation of indolines of the formula I

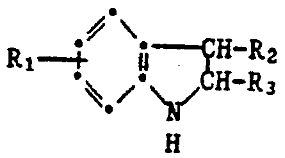

(I)

in which $R_1$ is hydrogen or $C_1$-$C_4$alkyl and each of $R_2$ and $R_3$ independently of the other is hydrogen, $C_1$-$C_4$alkyl or phenyl, by cyclodehydrating a 2-(2'-aminophenyl)ethanol of the formula II

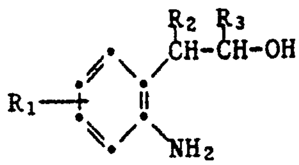

(II)

in which $R_1$, $R_2$ and $R_3$ are as defined for formula I, in the presence of a catalyst, which process comprises carrying out the cyclodehydration of the 2-(2'-aminophenyl)ethanol of formula II at 150°-350° C in the presence of an amorphous aluminosilicate (silica alumina).

2. A process according to claim 1, which comprises carrying out the cyclodehydration of the 2-(2'-aminophenyl)ethanol of formula II at 200°-300° C in the gaseous phase in the presence of an amorphous aluminosilicate (silica alumina) consisting of 5-30 % by weight of $Al_2O_3$ and 70-95 % by weight of $SiO_2$ and having a BET inner surface area of 50-800 $m^2/g$ and a pore volume of 0.1-1.0 $cm^3/g$.

3. A process according to claim 2, wherein the cyclodehydration of the 2-(2'-aminophenyl)ethanol of formula II is carried out at 240°-260° C.

4. A process according to claim 2, wherein the catalyst employed is an amorphous aluminosilicate consisting of 10-20 % by weight of $Al_2O_3$ and 80-90 % by weight of $SiO_2$ and having a BET inner surface area of 300-600 $m^2/g$ and a pore volume of 0.4-0.7 $cm^3/g$.

5. A process according to claim 4, wherein the catalyst employed is an amorphous aluminosilicate consisting of 10-15 % by weight of $Al_2O_3$ and 85-90 % by weight of $SiO_2$ and having a BET inner surface area of 300-500 $m^2/g$ and a pore volume of 0.5-0.7 $cm^3/g$.

6. A process according to claim 2, wherein the throughput of 2-(2'-aminophenyl)ethanol of formula II is 0.1 to 10 kg per kilogram of catalyst and per hour.

7. A process according to claim 6, wherein the throughput of 2-(2'-aminophenyl)ethanol of formula II is 0.5 to 2 kg per kilogram of catalyst and per hour.

8. A process according to claim 2, wherein the cyclodehydration of the 2-(2'-aminophenyl)ethanol of formula II is carried out in the presence of a carrier gas.

9. A process according to claim 8, wherein the carrier gas used is nitrogen, hydrogen or steam.

**10.** A process according to claim 8, wherein the carrier gas used is steam.

**11.** A process according to claim 8, wherein 1 to 10 moles of carrier gas are used per mole of 2-(2'-aminophenyl)ethanol of formula II.

**12.** A process according to claim 8, wherein 6 to 8 moles of carrier gas are used per mole of 2-(2'-aminophenyl)ethanol of formula II.

**13.** A process according to claim 2, which comprises cyclodehydrating 2-(2'-aminophenyl)ethanol.

**14.** A process according to claim 13, which comprises cyclodehydrating 2-(2'-aminophenyl)ethanol at 240°-260°C in the presence of an amorphous aluminosilicate consisting of 10-15 % by weight of $Al_2O_3$ and 85-90 % by weight of $SiO_2$ and having a BET inner surface area of 300-500 $m^2/g$ and a pore volume of 0.5-0.7 $cm^3/g$, as catalyst, and in the presence of 6 to 8 moles of steam per mole of 2-(2'-aminophenyl)ethanol, at a throughput of 2-(2'-aminophenyl)ethanol of 0.5 to 2 kg per kilogram of catalyst and per hour.

**15.** A process according to claim 1, which comprises carrying out the cyclodehydration of the 2-(2'-aminophenyl)ethanol of formula II at 150°-350°C in liquid phase in the presence of an amorphous alumino-silicate consisting of 5-30 % by weight of $Al_2O_3$ and 70-95 % by weight of $SiO_2$ and having a BET inner surface area of 50-800 $m^2/g$ and a pore volume of 0.1-1.0 $cm^3/g$.

**16.** A process according to claim 15, which comprises carrying out the cyclodehydration of the 2-(2'-aminophenyl)ethanol of formula II in the presence of an amorphous alumino silicate consisting of 10-20 % by weight of $Al_2O_3$ and 80-90 % by weight of $SiO_2$ and having a BET inner surface area of 300-600 $m^2/g$ and a pore volume of 0.4-0.7 $cm^3/g$.

**17.** A process according to claim 16, which comprises carrying out the cyclodehydration of the 2-(2'-aminophenyl)ethanol of formula II in the presence of an amorphous alumino silicate consisting of 10-15 % by weight of $Al_2O_3$ and 85-90 % by weight of $SiO_2$ and having a BET inner surface area of 300-500 $m^2/g$ and a pore volume of 0.5-0.7 $cm^3/g$.

**18.** A process according to claim 15, wherein the cyclodehydration of the 2-(2'-aminophenyl)ethanol of formula II is carried out at a temperature of 200°-280°C.

**19.** A process according to claim 15 which is carried out continuously or discontinuously.

**20.** A process according to claim 19 which is carried out continuously.

**21.** A process according to claim 15 which is carried out discontinuously and wherein the amorphous aluminosilicate is used in an amount of 0.5 to 10 % by weight, based on the 2-(2'-aminophenyl)ethanol of formula II.

**22.** A process according to claim 15 which is carried out continuously and wherein the amorphous aluminosilicate is used in an amount of 0.5 to 10 % by weight, based on the amount of reaction mixture present in the reactor.

**23.** A process according to claim 22, wherein the amorphous alumino silicate is used in an amount of 1-5 % by weight, based on the amount of reaction mixture present in the reactor.

**24.** A process according to claim 15, which comprises cyclodehydrating 2-(2'-aminophenyl)ethanol.

**25.** A process according to claim 24, which comprises cyclodehydrating 2-(2'-aminophenyl)ethanol in the presence of 1-5 % by weight, based on the amount of reaction mixture present in the reactor, of an amorphous aluminosilicate consisting of 10-15 % by weight of $Al_2O_3$ and 85-90 % by weight of $SiO_2$ and having a BET inner surface area of 300-500 $m^2/g$ and a pore volume of 0.5-0.7 $cm^3/g$ at an average residence time of 0.5-3 hours.

## Revendications

1. Procédé de préparation d'indolines de formule I

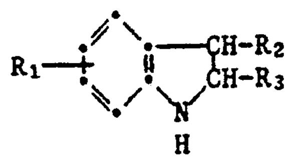

(I)

dans laquelle $R_1$ représente l'hydrogène ou un groupe alkyle en C1-C4 et $R_2$ et $R_3$ représentent chacun, indépendamment l'un de l'autre, l'hydrogène, un groupe alkyle en C1-C4 ou phényle, par cyclodéshydratation d'un 2-(2'-aminophényl)-éthanolde formule II

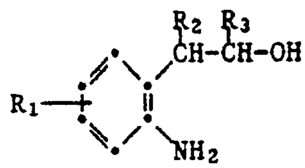

(II)

dans laquelle $R_1$, $R_2$ et $R_3$ ont les significations indiquées en référence à la formule I, en présence d'un catalyseur, caractérisé en ce que l'on procède à la cyclodéshydratation du 2-(2'-aminophényl)-éthanol de formule II à des températures de 150 à 350 °C en présence d'un aluminosilicate amorphe (silice-alumine).

2. Procédé selon la revendication 1, caractérisé en ce que l'on procède à la cyclodéshydratation du 2-(2'-aminophényl)-éthanol de formule II en phase gazeuse à des températures de 200 à 300 °C en présence d'un aluminosilicate amorphe (silice-alumine) contenant 5 à 30 % en poids d'$Al_2O_3$ et 70 à 95 % en poids de $SiO_2$, avec une surface interne, mesurée selon BET, de 50 à 800 $m^2/g$ et un volume de pores de 0,1 à 1,0 $cm^3/g$.

3. Procédé selon la revendication 2, caractérisé en ce que l'on procède à la cyclodéshydratation du 2-(2'-aminophényl)-éthanol de formule II à des températures de 240 à 260 °C.

4. Procédé selon la revendication 2, caractérisé en ce que l'on utilise en tant que catalyseur un aluminosilicate amorphe contenant 10 à 20 % en poids d'$Al_2O_3$ et 80 à 90 % en poids de $SiO_2$ avec une surface interne, mesurée selon BET, de 300 à 600 $m^2/g$ et un volume de pores de 0,4 à 0,7 $cm^3/g$.

5. Procédé selon la revendication 4, caractérisé en ce que l'on utilise en tant que catalyseur un aluminosilicate amorphe contenant de 10 à 15 % en poids d'$Al_2O_3$ et de 85 à 90 % en poids de $SiO_2$ avec une surface interne mesurée selon BET de 300 à 500 $m^2/g$ et un volume de pores de 0,5 à 0,7 $cm^3/g$.

6. Procédé selon la revendication 2, caractérisé en ce que l'on met en oeuvre de 0,1 à 10 kg de 2-(2'-aminophényl)-éthanol de formule II par kilogramme de catalyseur et par heure.

7. Procédé selon la revendication 6, caractérisé en ce que l'on met en oeuvre de 0,5 à 2 kg de 2-(2'-aminophényl)-éthanol de formule II par kilogramme de catalyseur et par heure.

8. Procédé selon la revendication 2, caractérisé en ce que l'on procède à la cyclodéshydratation du 2-(2'-aminophényl)-éthanol de formule II en présence d'un gaz véhicule.

9. Procédé selon la revendication 8, caractérisé en ce que l'on utilise en tant que gaz véhicule l'azote, l'hydrogène ou la vapeur d'eau.

10. Procédé selon la revendication 8, caractérisé en ce que l'on utilise en tant que gaz véhicule la vapeur

d'eau.

11. Procédé selon la revendication 8, caractérisé en ce que l'on utilise de 1 à 10 mol du gaz véhicule par mol du 2-(2'-aminophényl)-éthanol de formule II.

12. Procédé selon la revendication 8, caractérisé en ce que l'on utilise de 6 à 8 mol du gaz véhicule par mol du 2-(2'-aminophényl)-éthanol de formule II.

13. Procédé selon la revendication 2, caractérisé en ce que l'on soumet à cyclodéshydratation le 2-(2'-aminophényl)-éthanol.

14. Procédé selon la revendication 13, caractérisé en ce que l'on soumet le 2-(2'-aminophényl)-éthanol à cyclodéshydratation à des températures de 240 à 260 °C en présence d'un aluminosilicate amorphe contenant 10 à 15 % en poids d'$Al_2O_3$ et 85 à 90 % en poids de $SiO_2$, avec une surface interne, mesurée selon BET, de 300 à 500 $m^2/g$ et un volume de pores de 0,5 à 0,7 $cm^3/g$, servant de catalyseur, et en présence de 6 à 8 mol de vapeur d'eau par mol de 2-(2'-aminophényl)-éthanol, avec mise en oeuvre de 0,5 à 2 kg de 2-(2'-aminophényl)-éthanol par kilogramme de catalyseur et par heure.

15. Procédé selon la revendication 1, caractérisé en ce que l'on procède à la cyclodéshydratation du 2-(2'-aminophényl)-éthanol de formule II en phase liquide à des températures de 150 à 350 °C, en présence d'un aluminosilicate amorphe contenant 5 à 30 % en poids d'$Al_2O_3$ et 70 à 95 % en poids de $SiO_2$ avec une surface interne mesurée selon BET de 50 à 800 $m^2/g$ et un volume de pores de 0,1 à 1,0 $cm^3/g$.

16. Procédé selon la revendication 15, caractérisé en ce que l'on procède à la cyclodéshydratation du 2-(2'-aminophényl)-éthanol de formule II en présence d'un aluminosilicate amorphe contenant 10 à 20 % en poids d'$Al_2O_3$ et 80 à 90 % en poids de $SiO_2$, avec une surface interne mesurée selon BET de 300 à 600 $m^2/g$ et un volume de pores de 0,4 à 0,7 $cm^3/g$.

17. Procédé selon la revendication 16, caractérisé en ce que l'on procède à la cyclodéshydratation du 2-(2'-aminophényl)-éthanol de formule II en présence d'un aluminosilicate amorphe contenant 10 à 15 % en poids d'$Al_2O_3$ et 85 à 90 % en poids de $SiO_2$ avec une surface interne mesurée selon BET de 300 à 500 $m^2/g$ et un volume de pores de 0,5 à 0,7 $cm^3/g$.

18. Procédé selon la revendication 15, caractérisé en ce que l'on procède à la cyclodéshydratation du 2-(2'-aminophényl)-éthanol de formule II à une température de 200 à 280 °C.

19. Procédé selon la revendication 15, caractérisé en ce que l'on opère en continu ou en discontinu.

20. Procédé selon la revendication 19, caractérisé en ce que l'on opère en continu.

21. Procédé selon la revendication 15, caractérisé en ce que l'on opère en discontinu et en ce que l'aluminosilicate amorphe est utilisé en quantité de 0,5 à 10 % du poids du 2-(2'-aminophényl)-éthanol de formule II mis en oeuvre.

22. Procédé selon la revendication 15, caractérisé en ce que l'on opère en continu et en ce que l'aluminosilicate amorphe est mis en oeuvre en quantité de 0,5 à 10 % du poids du mélange de réaction présent dans le récipient de réaction.

23. Procédé selon la revendication 22, caractérisé en ce que l'on utilise l'aluminosilicate amorphe en quantité de 1 à 5 % du poids du mélange de réaction présent dans le récipient de réaction.

24. Procédé selon la revendication 15, caractérisé en ce que l'on soumet le 2-(2'-aminophényl)-éthanol à cyclodéshydratation.

25. Procédé selon la revendication 24, caractérisé en ce que l'on soumet le 2-(2'-aminophényl)-éthanol à cyclodéshydratation en présence de 1 à 5 % en poids d'un aluminosilicate amorphe contenant 10 à 15

% en poids d'$Al_2O_3$, 85 à 90 % en poids de $SiO_2$, avec une surface interne mesurée selon BET de 300 à 500 $m^2/g$ et un volume de pores de 0,5 à 0,7 $cm^3/g$, par rapport à la quantité du mélange de réaction présent dans le réacteur, avec une durée de passage moyenne de 0,5 à 3 heures.